(19) Europäisches Patentamt
European Patent Office
Office européen des brevets

(11) Veröffentlichungsnummer: **0 451 650 B1**

# (12) EUROPÄISCHE PATENTSCHRIFT

(45) Veröffentlichungstag der Patentschrift: **24.08.94**

(51) Int. Cl.$^{5}$: **C07C 45/36**, C07C 47/565

(21) Anmeldenummer: **91105110.0**

(22) Anmeldetag: **30.03.91**

## (54) Verfahren zur Herstellung von o-Hydroxy-benzaldehyden.

(30) Priorität: **13.04.90 DE 4012008**

(43) Veröffentlichungstag der Anmeldung:
**16.10.91 Patentblatt 91/42**

(45) Bekanntmachung des Hinweises auf die Patenterteilung:
**24.08.94 Patentblatt 94/34**

(84) Benannte Vertragsstaaten:
**AT BE CH DE DK FR GB IT LI NL SE**

(56) Entgegenhaltungen:
**EP-A- 0 012 939**
**EP-A- 0 330 036**
**DE-B- 1 128 847**

**PATENT ABSTRACTS OF JAPAN vol. 11, no. 365 (C-460)(2812) 27 November 1987,& JP-A-62 135443**

(73) Patentinhaber: **BAYER AG**

**D-51368 Leverkusen (DE)**

(72) Erfinder: **Schnatterer, Albert, Dr.**
**Walter-Flex-Strasse 32**
**W-5090 Leverkusen (DE)**
Erfinder: **Fiege, Helmut, Dr.**
**Walter-Flex-Strasse 23**
**W-5090 Leverkusen (DE)**
Erfinder: **Neumann, Karl-Heinz, Dr.**
**Vilicher-Strasse 81**
**W-5205 St. Augustin 2 (DE)**

**EP 0 451 650 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von o-Hydroxy-benzaldehyden durch katalytische Oxidation der zugrundeliegenden o-Kresole mit Sauerstoff in Gegenwart von Chelatkomplexen des Eisens, Mangans oder eines Gemisches beider.

o-Hydroxy-benzaldehyde sind wichtige Zwischenprodukte für industrielle Synthesen von Riechstoffen, Farbstoffen, Pharmazeutika, Pflanzenschutzmitteln und Fotochemikalien. Der industriell wichtigste Vertreter ist der Salicylaladehyd. Er dient u.a. als Ausgangsmaterial für die Herstellung von Cumarin.

Salicylaldehyd ist technisch auf mehreren Wegen zugänglich. Ausgangsmaterial ist entweder Phenol oder o-Kresol (Ullmanns Encyklopädie der Technischen Chemie, 5. Aufl., Band A3 (1985), S. 470). Bei der Reimer-Tiemann-Reaktion von Phenol mit Chloroform in Gegenwart von Alkali entsteht ein Isomerengemisch aus o-Hydroxy- und p-Hydroxy-benzaldehyd (US-3 365 500, EP-68 725). Die Bildung des p-Hydroxy-Isomeren wird vermieden durch ein Verfahren, bei welchem in einem ersten Schritt Phenol in Form des Borsäureesters mit Formaldehyd selektiv in der o-Stellung hydroxymethyliert wird. Nach der Spaltung des Borsäureesters wird der o-Hydroxy-benzylalkohol in einem weiteren Schritt mit Sauerstoff in flüssiger Phase an einem Platinkontakt zu Salicylaldehyd oxidiert (DE-AS 12 61 517, DE-OS 26 12 844). Von o-Kresol ausgehend kann Salicylaldehyd durch Seitenkettenchlorierung und Verseitung der Dichlormethylgruppe zur Aldehydgruppe hergestellt werden. Die phenolische OH-Gruppe muß bei diesem Prozeß durch Veresterung geschützt werden (JP-73/03831, zitiert nach C.A. 79 (1973), 18387x). Weitere Verfahren beruhen auf der elektrochemischen Reduktion von Salicylsäure (Ind. Chemist 39 (1963), 238-41, zitiert nach C.A. 59 (1963), 10986b) und der katalytischen Reduktion von Salicylsäurehalogeniden (JP-68/13204, zitiert nach C.A. 70-(1969), 28646j).

Aus EP-A-330 036 ist bekannt, daß p-Hydroxy-benzaldehyde durch Oxidation der zugrundeliegenden p-Kresole mit Sauerstoff in Gegenwart basisch reagierender Stoffe in einem Lösungsmittel und in Gegenwart eines Chelatkomplexes von Eisen und/oder Mangan erhalten werden können.

Die bisherigen Verfahren haben den Nachteil, daß sie entweder Isomerengemische liefern oder aber aufwendige mehrstufige Verfahren darstellen, bei denen beispielsweise Hilfsstoffe wie Borsäure oder Hilfsstoffe zum Schutz reaktiver funktioneller Gruppen an den umzusetzenden Stoffen benötigt werden.

Nach EP- 12 939 lassen sich p-Kresolderivate mit Sauerstoff in flüssiger Phase in Gegenwart einer Base und einer Kobaltverbindung in brauchbaren Ausbeuten zu den entsprechenden p-Hydroxy-benzaldehyden oxidieren. Diese Schrift weist gleichzeitig darauf hin, daß o-Kresol auf diese Weise nicht oxidiert werden kann.

Es wurde ein Verfahren zur Herstellung von o-Hydroxy-benzaldehyden der Formel

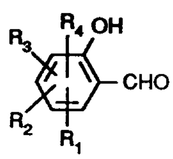

(I),

in der

$R_1$, $R_2$, $R_3$ und $R_4$ unabhängig voneinander Wasserstoff, geradkettiges oder verzweigtes $C_1$-$C_{10}$-Alkyl, $C_3$-$C_8$-Cycloalkyl, geradkettiges oder verzweigtes $C_1$-$C_{10}$-Alkoxy, Phenyl oder Halogen bedeuten, wobei in p-Stellung zur Hydroxylgruppe Alkyl oder Cycloalkyl nur stehen dürfen, wenn sie keine $\alpha$-H-Atome tragen,

durch katalytische Oxidation von o-Kresolen der Formel

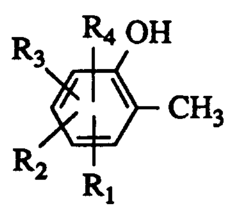

(II),

in der

$R_1$-$R_4$ die angegebene Bedeutung haben,

mit Sauerstoff in Gegenwart basisch reagierender Stoffe in einem Lösungsmittel gefunden, das dadurch gekennzeichnet ist, daß als Katalysatoren Chelatkomplexe des Eisens, Mangans oder eines Gemisches beider mit Polyaza-Makrocyclen als Chelatbildner eingesetzt werden.

Das erfindungsgemäße Verfahren bringt gegenüber dem Stand der Technik einen entscheidenden Fortschritt, da es die selektive Herstellung von o-Hydroxy-benzaldehyden aus den zugrundeliegenden o-Kresolen in einem Reaktionsschritt erlaubt. o-Kresole sind technisch leicht zugangliche Verbindungen. Die Verwendung von Sauerstoff als Oxidationsmittel stellt sowohl, was den Preis als auch die Ökologie anbelangt, ein Optimum dar.

Die selektive Oxidation der zur Hydroxylgruppe o-ständigen Methylgruppe bleibt auf der Stufe der Aldehydgruppe stehen; eine Weiteroxidation zur Carboxylgruppe wird nicht beobachtet. Das erfindungsgemäße Verfahren ist umso überraschender, als die bekannten Verfahren zur selektiven Oxidation von p-Kresol zu p-Hydroxy-benzaldehyd mit Sauerstoff sich nicht auf o-Kresole anwenden lassen (EP-12 939). Befindet sich in der zweiten o-Position zur Hydroxylgruppe eine weitere Methylgruppe, wird auch diese zur Aldehydgruppe oxidiert. Methylgruppen in m-Stellung zur Hydroxylgruppe werden unter den erfindungsgemäßen Reaktionsbedingungen nicht angegriffen.

$C_1$-$C_{10}$-Alkyl kann geradkettig oder verzweigt sein und ist beispielsweise Methyl, Ethyl, Propyl, Isopropyl, Butyl, Isobutyl, tert.-Butyl, eines der isomeren Pentyle, Hexyle, Octyle oder Decyle.

$C_3$-$C_8$-Cycloalkyl kann außer den Ring-C-Atomen noch C-Atome in Form von 1 oder 2 Methyl- oder Ethylsubstituenten aufweisen und ist beispielsweise Cyclopropyl, Methyl-cyclopropyl, Dimethyl-cyclopropyl, Cyclobutyl, Cyclopentyl, Methyl-cyclopentyl, Dimethyl-cyclopentyl, Cyclohexyl, Methyl-cyclohexyl, Dimethyl-cyclohexyl, Cycloheptyl oder Cyclooctyl sowie entsprechendes mit Ethyl substituiertes Cycloalkyl.

$C_1$-$C_{10}$-Alkoxy kann geradkettig oder verzweigt sein und ist beispielsweise Methoxy, Ethoxy, Propoxy, Isopropoxy, Butoxy, Isobutoxy, tert.-Butoxy, eines der isomeren Pentyloxy, Hexyloxy, Octyloxy oder Decyloxy.

Halogen ist beispielsweise Fluor, Chlor oder Brom, bevorzugt Chlor oder Brom.

Alkyl und Alkoxy haben in bevorzugter Weise 1-6 C-Atome, in besonders bevorzugter Weise 1-4 C-Atome. Cycloalkyl hat in bevorzugter Weise 3,5 oder 6 C-Atome, in besonders bevorzugter Weise 5 oder 6 C-Atome und kann ein- oder zweifach durch Methyl oder Ethyl substituiert sein.

Phenyl kann durch Halogen der genannten Art und/oder durch $C_1$-$C_4$-Alkyl der genannten Art ein- oder zweifach substituiert sein.

In bevorzugter Weise werden o-Kresole der Formel

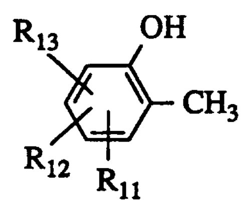

(III)

eingesetzt, in der

$R_{11}$, $R_{12}$ und $R_{13}$ unabhängig voneinander Wasserstoff, geradkettiges oder verzweigtes $C_1$-$C_6$-Alkyl, $C_5$-$C_6$-Cycloalkyl, geradkettiges oder verzweigtes $C_1$-$C_6$-Alkoxy, Phenyl oder Halogen bedeuten, wobei in p-Stellung zur Hydroxylgruppe Alkyl oder Cycoalkyl nur stehen dürfen, wenn sie keine $\alpha$-H-Atome tragen.

In besonders bevorzugter Weise werden o-Kresole der Formel

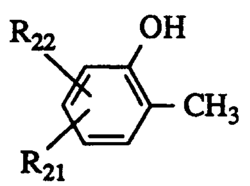

(IV)

eingesetzt, in der

$R_{21}$ und $R_{22}$ unabhängig voneinander für Wasserstoff, geradkettiges oder verzweigtes $C_1$-$C_6$-Alkyl,

Cyclohexyl, geradkettiges oder verzweigtes $C_1$-$C_6$-Alkoxy, Phenyl oder Halogen stehen, wobei in p-Stellung zur Hydroxylgruppe Alkyl oder Cyclohexyl nur stehen dürfen, wenn sie keine α-H-Atome tragen.

Alkyl oder Cycloalkyl, die in p-Stellung zur Hydroxylgruppe stehen, tragen erfindungsgemäß keine α-H-Atome. Dies ist dann gegeben, wenn das urmittelbar mit dem aromatischen Kern verknüpfte C-Atom ein tertiäres ist; Beispiele hierfür sind die tert.-Butylgruppe und die 1-Methyl-cyclohexylgruppe.

Aus der Vielzahl der erfindungsgemäß einsetzbaren o-Kresole seien beispielhaft genannt: o-Kresol, 4-Chlor-2-methylphenol, 4,6-Dibrom-2-methylphenol, 2,6-Dimethylphenol, 2,3-Dimethylphenol; besonders wichtig ist das erfindungsgemäße Verfahren für die Oxidation von o-Kresol.

Das erfindungsgemäße Verfahren ist gekennzeichnet durch den Einsatz von Chelatkomplexen des Eisens, Mangans oder eines Gemisches beider mit Polyaza-Makrocyclen als Chelatbildner. Wichtige Vertreter dieser Polyaza-Makrocyclen sind die Aza-Annulene. Als Beispiel eines erfindungsgemäß eissetzbaren Polyaza-Annulens sei das 5,14-Dihydro[b,i][5,9,14,18]tetraaza[14]annulen genannt (Liebigs Ann. Chem. 717(1968), 137-147).

Besonders wichtige und daher bevorzugte Polyaza-Makrocyclen sind Porphyrine, Phthalocyanine, porphyrinanaloge Verbindungen und solche Verbindungen, die aus diesen durch chemische Modifizierung hervorgehen, beispielsweise die Chlorine als teilhydrierte Porphyrine. Die Porphyrine können hierbei natürlichen Ursprungs sein, z.B. Härmatoporphyrin; sie können jedoch auch synthetischer Art sein. In bevorzugter Weise wird das Eisen, Mangan oder ein Gemisch beider in Form von Komplexen mit synthetischen Makrocyclen eingesetzt. Diese Metallkomplexe werden durch die Formel

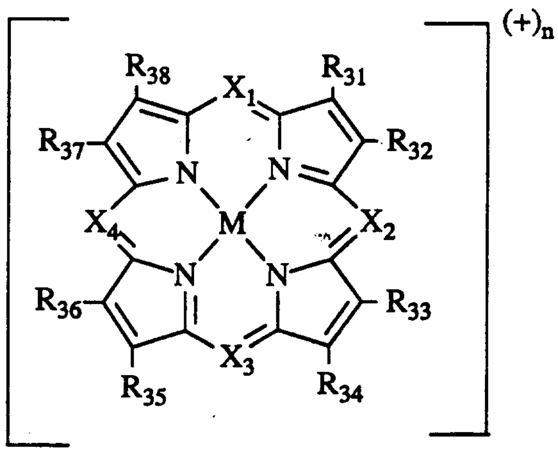

$(X^-)_n$ (V)

repräsentiert, in der

M Eisen oder Mangan bedeutet,

$X_1$, $X_2$, $X_3$ und $X_4$ unabhängig für N oder für das mit dem gleichen Index gekennzeichnete $CY_1$, $CY_2$, $CY_3$ bzw. $CY_4$ stehen, wobei $Y_1$, $Y_2$, $Y_3$ und $Y_4$ unabhängig voneinander Wasserstoff, geradkettiges oder verzweigtes $C_1$-$C_{10}$-Alkyl, $C_3$-$C_8$-Cycloalkyl, $C_7$-$C_{10}$-Aralkyl, $C_6$-$C_{14}$-Aryl, welches teilhydriert sein kann, oder ein aromatisches oder nichtaromatisches 5- oder 6-gliedriges heterocyclisches Ringsystem mit 1 bis 3 Heteroatomen aus der Gruppe N, O und S bedeuten,

$R_{31}$, $R_{32}$, $R_{33}$, $R_{34}$, $R_{35}$, $R_{36}$, $R_{37}$ und $R_{38}$ unabhängig voneinander Wasserstoff, Halogen, geradkettiges oder verzweigtes $C_1$-$C_{10}$-Alkyl, $C_3$-$C_8$-Cycloalkyl, $C_7$-$C_{10}$-Aralkyl oder Phenyl bedeuten, wobei die beiden Reste eines Pyrrolringes gemeinsam mit den durch sie substituierten C-Atomen $C_6$-$C_{14}$-Aryl, welches teilhydriert sein kann, oder ein aromatisches oder nicht aromatisches 5- oder 6-gliedriges heterocyclisches Ringsystem mit 1 bis 3 Heteroatomen aus der Gruppe N, O und S bilden können,

$X^-$ ein Anionäquivalent einer anorganischen oder organischen Säure oder Hydroxyl oder Alkoxy ist und

n die Zahl Null oder Eins bedeutet.

Geradkettiges oder verzweigtes $C_1$-$C_{10}$-Alkyl, $C_3$-$C_8$-Cycloalkyl und Halogen haben den obengenannten Bedeutungsumfang. $C_7$-$C_{10}$-Aralkyl ist beispielsweise Benzyl, Phenyl-ethyl, Phenyl-propyl oder Phenylbutyl, bevorzugt Benzyl. $C_6$-$C_{14}$-Aryl ist beispielsweise Phenyl, Naphthyl, Anthryl, Phenanthryl oder Biphenylyl, bevorzugt Phenyl oder Naphthyl. Ein solches Aryl umfaßt definitionsgemäß auch die durch Teilhydrierung daraus hervorgegangenen Ringsysteme. Man gelangt so im Falle der an den Pyrrolkernen sitzenden Reste $R_{31}$ bis $R_{38}$ vom Pyrrolsystem beispielsweise zum Isoindolsystem (3,4-benzokondensiertes Pyrrol), zum 3,4-Naphthaleno-pyrrol, zum 3,4-Anthraceno-pyrrol, aber auch zum Cyclohexano-pyrrol oder zum Cyclohexadieno-pyrrol als Beispiele für aromatisches bzw. teilhydriertes Aryl.

Wichtige Beispiele für heterocyclische Ringsysteme als Substituenten, die auch benzokondensiert sein können, sind: Indolyl, Chinolyl, Isochinolyl, Pyrazolyl, Triazolyl, Thiazolyl, Oxazolyl, Pyrimidinyl, Imidazolyl, Carbazolyl, Pyridyl, Thienyl, Pyrazinyl.

Die genannten Substituenten, insbesondere die aromatischen Substituenten, können je nach Anzahl von substituierbaren H-Atomen 1 bis 5fach, bevorzugt 1 bis 3fach substituiert sein, beispielsweise durch geradkettiges oder verzweigtes $C_1$-$C_6$-Alkyl, $C_1$-$C_6$-Alkoxy, $C_1$-$C_6$-Alkylthio, durch Hydroxy, Mercapto, Halogen, Carboxyl, Sulfonyl, Nitro, Cyano, Amino, $C_1$-$C_6$-Alkyl-amino und Di-($C_1$-$C_6$-alkyl)-amino, wobei die Alkylreste ihrerseits durch Halogen, $C_1$-$C_4$-Alkoxy, $C_1$-$C_4$-Alkylthio, Amino, Alkylamino, Hydroxy, Mercapto, Carboxyl, Sulfonyl, Nitro substituiert sein können.

$X^-$ als Anionäquivalent unterliegt keiner spezifischen Beschränkung; Beispiele sind: Fluorid, Chlorid, Bromid, Iodid, Azid, Nitrit, Nitrat, Methoxy, das Acetatanion, bevorzugt Chlorid, Bromid, Hydroxy, das Acetanion.

Sind $X_1$ bis $X_4$ gleich und bedeuten $CY_1$, $CY_2$, $CY_3$ bzw. $CY_4$, wobei $Y_1$ bis $Y_4$ die oben angegebene Bedeutung haben, bezeichnet man die Verbindungen der Formel (V) allgemein als Porphyrine. Eine große Anzahl von Porphyrinen ist durch Kondensation von Aldehyden mit substituiertem oder unsubstituiertem Pyrrol synthetisiert worden. Beispielsweise wird durch Kondensation von Benzaldehyd mit Pyrrol meso-Tetaphenylporphin (J. Org. Chem. 32, 476 (1967)) erhalten. Auf ähnliche Weise erhält man meso-Tetra-n-propyl-porphin durch Kondensation von Pyrrol mit n-Butyraldehyd oder Octamethyl-porphin durch Umsetzung von Formaldehyd mit 3,4-Dimethylpyrrol (Liebigs Ann. Chem. 718 (1968) 183-207). Wird die Kondensation von Aldehyd und Pyrrol mit einem Gemisch aus verschiedenen Aldehyden und verschieden 3,4-substituierten Pyrrolen durchgeführt, kann man zu solchen Makrocyclen der Formel (V) gelangen, in denen sowohl $X_1$ bis $X_4$ als auch die vier Pyrrolringe unterschiedlich substituiert sein können. Normalerweise fallen bei einer solchen Synthese Porphyringemische an. Diese Gemische können direkt für erfindungsgemäße einsetzbare Chelate verwendet werden. Es können aber auch die Einzelkomponenten nach deren Auftrennung eingesetzt werden. Die Metallierung der bei diesen Synthesen anfallenden Porphyrine erfolgt durch Umsetzung mit Eisen- bzw. Mangansalzen, beispielsweise in Dimethylformamid (J. Inorg. Nucl. Chem. 32-(1970), 2443).

Sind in der Formel (V) $X_1$ bis $X_4$ gleich und bedeuten $CY_1$, $CY_2$, $CY_3$ bzw. $CY_4$, wobei $Y_1$ bis $Y_4$ die oben angegebene Bedeutung haben, und sind die beiden Reste jedes Pyrrolrings Teil eines Carbocyclus oder Heterocyclus, so beschreibt die Formel (V) eine Klasse von Porphyrinen, deren Grundkörper das Benzoporphyrin ist, bei dem $X_1$ bis $X_4$ CH bedeuten und jeder Pyrrolring benzokondensiert ist. Mögliche Herstellungswege für Benzoporphyrin sind die Selbstkondensationen von Methylphthalimidin oder o-Cyanacetophenon in Gegenwart von Magnesium (Liebigs Ann. Chem. 533(1938), 197). Zum gewünschten Eisen- bzw. Mangan-benzoporphyrin kann man prinzipiell durch ein geeignetes Ummetallierungsverfahren kommen. Durch analoge Kondensation substituierter o-Cyan-arylketone ist prinzipiell eine große Vielfalt weiterer Porphyrine zugänglich. Solche Porphyrine können auch zusätzlich meso-substituiert sein.

Bedeuten in der Formel (V) einzelne oder alle von $X_1$ bis $X_4$ N, so werden die Verbindungen je nach Anzahl der Azagruppen als Mono-, Di-, Tri- und Tetraazaporphyrine oder allgemein als Azaporphyrine bezeichnet. Ein Sonderfall ist das als Phthalocyanin bekannte Tetrabenzo-tetraazaporphyrin. Herstellungsmöglichkeiten für Azaporhyrine und Phthalocyanine beispielsweise Phthalocyanin, Phthalocyanintetrasulfonat, Naphthalocyanin, Tetrathiophenotetraazaporphin, Octamethyltetraazaporphin können der Literatur entnommen werden. Mono-, Di- und Triazaporphyrine sind auf ähnlichen Wegen zugänglich wie die Benzoporphyrine und Tetraazaporphyrine. Mono- und Di-azaporphyrine können beispielsweise durch Erhitzen eines Gemisches von Phthalsäuredinitril, o-Cyan-arylketon und Metallsalz nach Liebigs Ann. Chem. 531 (1937) 279 erhalten werden, z.B. der Eisen(II)-Komplex des Mono- und Di-azatetrabenzoporphyrins. Tri-azaporphyrine können beispielsweise durch Erhitzen eines Gemisches aus Phthalsäuredinitril, Methylenphthalimiden und Metallsalz hergestellt werden (EP 0 003 149). Durch geeignete Substitution am Phthalsäuredinitril oder am o-Cyan-arylketon ist nach diesen Verfahren eine große Vielfalt von Verbindungen der Formel (V) zugänglich, darunter auch solche, die zusätzlich meso-substituiert sind.

In besonders bevorzugter Weise werden meso-Tetraarylporphine eingesetzt. Die Eisen- und Mangankomplexe der erfindungsgemäß einsetzbaren meso-Tetraarylporphine werden durch die Formel

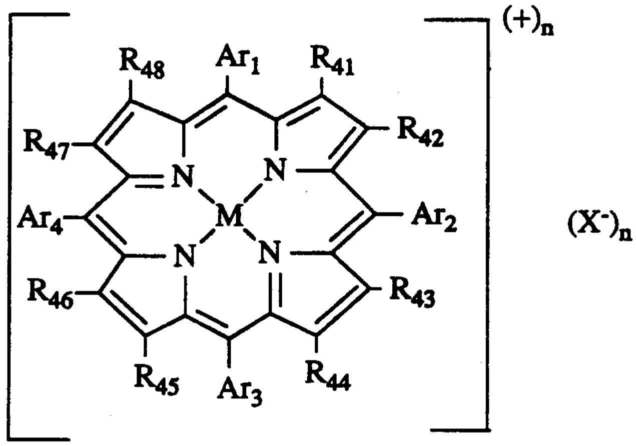

(VI)

repräsentiert, wobei

| | |
|---|---|
| M, $X^-$ und n | die obige Bedeutung haben, |
| $R_{41}$, $R_{42}$, $R_{43}$, $R_{44}$, $R_{45}$, $R_{46}$, $R_{47}$ und $R_{48}$ | unabhängig voneinander Wasserstoff, Halogen oder $C_1$-$C_4$-Alkyl bedeuten und jeweils einer von $R_{41}$ bis $R_{48}$ pro Pyrrolkern auch $C_7$-$C_{10}$-Aralkyl oder Phenyl sein kann und |
| $Ar_1$, $Ar_2$, $Ar_3$ und $Ar_4$ | unabhängig voneinander ein carbocyclischer oder heterocyclischer aromatischer Rest sind. |

Porphyrine der Formel (VI) lassen sich beispielsweise durch Kondensation von unsubstituiertem oder geeignet substituiertem Pyrrol und entsprechend substituierten Arylaldehyden metallfrei herstellen (J. Org. Chem. 32 (1967), 476). Die Metallierung erfolgt durch Umsetzung mit Eisen- bzw. Mangansalzen, beispielsweise in Dimethylformamid (J. Inorg. Nucl. Chem. 32 (1970), 2443). Werden bei der Kondensation von Pyrrol und Arylaldehyd Gemische aus unterschiedlich substituierten Pyrrolen und Gemische aus verschiedenen Arylaldehyden eingesetzt, so gelangt man zu Porphyrinen der Formel (VI), die sowohl an den einzelnen meso-Positionen als auch am Pyrrolteil jeweils unterschiedlich substituiert sein können. Solche Porphyrine fallen normalerweise als Gemische mit mehreren Porphyrinen verschiedener Zusammensetzung an. Diese Porphyringemische können für das erfindungsgemäße Verfahren direkt eingesetzt werden.

Beispiele für erfindungsgemäß einsetzbare meso-Tetraarylporphine, die Metallkomplexe der Formel (VI) bilden, sind:

Tetraphenylporphin
Tetrakis-(2-methoxyphenyl)-porphin
Tetrakis-(2,4-dimethoxyphenyl)-porphin
Tetrakis-(2,3,4-trimethoxyphenyl)-porphin
Tetrakis-(2-ethoxyphenyl)-porphin
Tetrakis-(2-isopropoxyphenyl)-porphin
Tetrakis-(2-bromphenyl)-porphin
Tetrakis-(2,4-dimethylphenyl)-porphin
Tetrakis-(2,6-dichlorphenyl)-porphin
Tetrakis-(pentafluorphenyl)-porphin
Tetrakis-(2-mercaptomethylphenyl)-porphin
Tetrakis-(2-pyridyl)-porphin
Tetrakis-(2-methylpyridinium)-porphin
Tetrakis-(4-methylpyridinium)-porphin
Tetrakis-(4-dimethylamino-2-methoxyphenyl)-porphin
Tetrakis-(2-aminophenyl)-porphin
Tetrakis-(2-phenyl-trimethylamonium-chlorid)-porphin
Tetrakis-(2-carboxyphenyl)-porphin
Tetrakis-(2-phenylsulfonsäure-natrium)-porphin
Tetrakis-[naphthyl(1)]-porphin
Tetrakis-[2-methoxy-naphthyl(1)]-porphin

Tetrakis-[anthracenyl(9)]-porphin
Tetrakis-[3-methylthiophenyl(2)]-porphin
Tetrakis-(2-hydroxyphenyl)-porphin
Tetrakis-(2-trifluormethyl)-porphin
Tetrakis-(2-hydroxy-4-methoxyphenyl)-porphin
Tetrakis-(4-dimethylamino-2-hydroxyphenyl)-porphin
Tetrakis-(2-mercaptophenyl)-porphin
Tetrakis-(2-hydroxy-5-nitrophenyl)-porphin
Tetrakis-[2-(2-hydroxyethoxy)-phenyl]-porphin
Tetrakis-[2-(2-mercaptoethoxy)-phenyl]-porphin
Tetrakis-[2-(2-methylmercaptoethoxy)-phenyl]-porphin
Tetrakis-[2-(2-carboxyethoxy)-phenyl]-porphin
meso-Tetrakis-(pentafluorphenyl)-oktafluorporphin
meso-Tetraphenyl-octamethyl-porphin
Tetrakis-[8-hydroxy-4-methoxy-naphthyl(1)]-porphin
Tetrakis-[8-hydroxy-chinolinyl-(7)]-porphin
Tetrakis-[8-methoxy-chinolinyl(7)]-porphin

Unter den meso-Tetraaryl-porphinen der Formel (VI) sind solche bevorzugt, deren Arylteil mindestens in der 2-Stellung substituiert ist. Ein solcher Substituent in 2-Stellung kann beispielsweise auch ein ankondensierter Carbocyclus oder Heterocyclus sein.

Beispiele für diese bevorzugten meso-Tetraarylporphine sind:

Tetrakis-(2-methoxyphenyl)-porphin
Tetrakis-(2-hydroxyphenyl)-porphin
Tetrakis-(2,4-dimethoxyphenyl)-porphin
Tetrakis-(2,3-dimethoxyphenyl)-porphin
Tetrakis-(2-bromphenyl)-porphin
Tetrakis-(2-ethoxyphenyl)-porphin
Tetrakis-(2-fluorphenyl)-porphin
Tetrakis-[8-hydroxy-chinolinyl(7)]-porphin
Tetrakis-(4-dimethylamino-2-hydroxyphenyl)-porphin

In einer dem Fachmann geläufigen Weise können solche Koordinationsstellen am Metall, die nicht durch den Komplexliganden besetzt sind, durch beliebige Donoren besetzt werden, beispielsweise durch Ammoniak, Kohlenmonoxid, Amine, wie Pyridin, Piperidin, Imidazol, Diethylamin, durch Sulfide, wie Dimethylsulfid oder Dimethylsulfoxid oder durch Phosphorverbindungen, wie Tributylphosphin oder Tributylphosphit. Solche Donoren können auch getrennt vom Eisen- und/oder Mangankomplex in das Reaktionsgemisch gegeben werden.

Die erfindungsgemäßen Eisen- und/oder Mangankomplexe können auch als Dimere, Oligomere oder Polymere eingesetzt werden. Die Dimerisierung, Oligomerisierung oder Polymerisierung kann beispielsweise durch Brückenbildung zwischen den Metallzentren oder durch Vernetzung über die Komplexliganden bewirkt werden. Beispiele für den Einsatz der erfindungsgemäßen Komplexe als Dimere sind die Oxoverbrückten Metall-Tetraaryl-Porphine $(FeTAP)_2O$ und $(MnTAP)_2O$ (TAP = Tetraarylporphin). Der Einsatz in polymerer Form kann beispielsweise nach Vernetzung mit einem geeignet funktionalisierten Polystyrol erfolgen (J. Macromol. Sci. - Chem., A 25 (1988), 1127-36). Weiterhin kann der Eisen- und/oder Mangankomplex auch in Kombination mit einem oberflächenaktiven Stoff, beispielsweise in Kombination mit Aktivkohle, eingesetzt werden.

Die erfindungsgemäß eingesetzten Eisen- und/oder Mangankomplexe sind erstaunlicherweise bereits in Spuren hochwirksam. Das molare Verhältnis von Eisen- und/oder Mangankomplex zu eingesetztem o-Kresol beträgt 0,000001-0,05:1, bevorzugt 0,00001-0,01:1. Selbstverständlich kann eine größere Menge an Chelatkomplex als die angegebene eingesetzt werden, jedoch bringt dies keinen zusätzlichen Vorteil, sondern erhöht lediglich die Kosten und kann unter Umständen die Aufarbeitung erschweren.

Vorteilhaft, aber nicht notwendig, werden im erfindungsgemäßen Verfahren die Eisen- und/oder Mangankomplexe in Kombination mit Kupfer oder einer Kupferverbindung als Co-Katalysator eingesetzt. Das Kupfer kann in den Co-Katalysatoren die Wertigkeiten 0, 1, 2 oder 3, bevorzugt 1 oder 2 annehmen. Mögliche Einsatzformen sind die Kupfersalze anorganischer Säuren, beispielsweise Kupferfluorid, Kupferchlorid, Kupferbromid, Kupferiodid, Kupfersulfat, Kupfernitrat, basisches Kupfercarbonat, Kupfercyanid, Kupferphosphat, Kupferborat: die Kupferoxide; die Kupfersalze organischer Säuren, wie Kupferacetat, Kupferoxalat, Kupfercitrat, Kupferstearat; Ionenaustauscher, die Kupfer enthalten; Kupferkomplexe, wie Kupferacetylacetonat, N,N'-Disalicylidenethylendiamin-kupfer(II). Auch Kupfersalze können in Kombination

mit einem komplexierenden Agens eingesetzt werden; komplexierende Agentien sind beispielsweise Ammoniak, Amine, wie Ethylendiamin und Sulfide, wie Dimethylsulfid. Selbst der Einsatz von elementarem Kupfer ist wirksam; wie oben bereits beschrieben. In bevorzugter Form werden Kupfersalze, sowohl in hydratisierter als auch in wasserfreier Form, oder Kupferoxid eingesetzt, beispielsweise $CuCl_2.2H_2O$, $Cu(NO_3)_2.3H_2O$, $Cu(CH_3COO)_2.H_2O$, $CuSO_4.5H_2O$, CuO, CuCl.

Auch Kupfer bzw. Kupferverbindungen als Co-Katalysatoren entfalten ihre co-katalytische Aktivität bereits in sehr kleiner Konzentration Kupfer oder eine Kupferverbindung wird in einem molaren Verhältnis zum o-Kresol von 0,000001-0,1:1, bevorzugt 0,00001-0,05:1 eingesetzt.

Als basisch reagierende Stoffe sind im erfindungsgemäßen Verfahren alle geeignet, die eine höhere Basizität aufweisen als die daraus herstellbaren o-Kresolate, beispielsweise Metallhydroxide, Metallalkoholate und Metallamide von Alkali- und Erdalkalimetallen und, soweit es sich um Alkoholate und Amide handelt, von Aluminium. Wichtige Metalle unter den genannten sind beispielsweise Natrium, Kalium, Lithium, Calcium, Magnesium. Als Alkoholate seien beispielsweise das Methylat, das Ethylat, das Isopropylat und das tert.-Butylat aufgeführt. Die Amide können beispielsweise Diethylamid, Ethylamid, Diisopropylamid, Dibutylamid usw. sein. Unter den genannten Basen sind die Hydroxide und die Alkoholate bevorzugt; insbesondere bevorzugt sind Natriumhydroxid, Kaliumhydroxid, Natriummethylat, Natriumethylat und Kalium-tert.-butylat.

Der basisch reagierende Stoff wird in einer Menge von 1-10 Basenäquivalenten pro Mol des o-Kresols eingesetzt; in bevorzugter Weise werden 1,5-6 Basenäquivalente eingesetzt.

Als Lösungsmittel für das erfindungsgemäße Verfahren kommen solche in Frage, die unter den Reaktionsbedingungen nicht oder nur sehr langsam mit Sauerstoff reagieren und die zu oxidierenden o-Kresole lösen. Solche Lösungsmittel sind beispielsweise Alkohole, Ether, Kohlenwasserstoffe, halogenierte Kohlenwasserstoffe, Amine, Amine, Sulfoxide. Solche Lösungsmittel können einzeln oder in Gemischen von 2 oder mehreren Lösungsmitteln eingesetzt werden. Sofern diese Lösungsmittel ganz oder teilweise mit Wasser mischbar sind, kommen auch solche Mischungen mit Wasser in Frage. In bevorzugter Weise werden alkoholische Lösungsmittel eingesetzt, wie Methanol, Ethanol, Isopropanol, Butanol, tert.-Butanol und Ethylenglykol. Ganz besonders bevorzugt ist Methanol als Lösungsmittel.

Neben den o-Hydroxy-benzaldehyden (I) findet man im Reaktionsgemisch gegebenenfalls substituierte o-Hydroxy-benzylalkohole der Formel

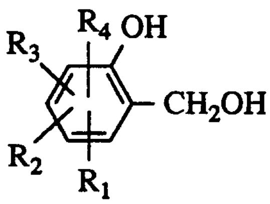

(VII)

und für den Fall der Verwendung von Alkoholen als Lösungsmittel auch o-Hydroxy-benzylalkylether der Formel

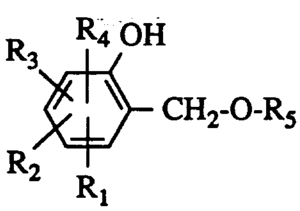

(VIII),

worin

$R_1$ bis $R_4$ den oben angegebenen Bedeutungsumfang haben und

$R_5$ der Alkylrest des als Lösungsmittel verwendeten Alkohols ist.

$R_5$ ist beispielsweise $C_1$-$C_4$-Alkyl der obengenannten Art, bevorzugt Methyl.

Die Menge dieser Alkohole (VII) und Ether (VIII) schwankt etwas mit den Reaktionsbedingungen. Beide Stoffgruppen können in einen Folgeansatz recyclisiert werden und erfahren darin eine Weiteroxidation zu den gewünschten o-Hydroxy-benzaldehyden.

Als Sauerstoff für das erfindungsgemäße Verfahren kann reiner Sauerstoff oder Sauerstoff in verdünnter Form, beispielsweise in Form von Sauerstoff enthaltenden Gasen, eingesetzt werden. Die wirtschaftlich günstigste Form des erfindungsgemäß verwendeten Sauerstoffs ist die atmosphärische Luft. Der Druck des Sauerstoffs bzw. des Sauerstoff enthaltenden Gases ist keiner besonderen Einschränkung unterworfen und kann zwischen 0,5 und 20 bar liegen, vorzugsweise bei 0,8-10 bar, besonders bevorzugt in der Nähe des atmosphärischen Druckes. Der Sauerstoffgehalt ist bei Verwendung von Sauerstoff enthaltenden Gasen ebenfalls keiner Beschränkung unterworfen. Der Sauerstoffgehalt richtet sich vor allem nach betrieblichen Gesichtspunkten, wie der Vermeidung explosionsfähiger Gemische mit den Lösungsmitteldämpfen. Der Gehalt und die Menge an Sauerstoff richtet sich weiterhin nach der Umsetzungsgeschwindigkeit. Es ist von Vorteil, den Sauerstoff im Reaktionsgemisch fein zu verteilen, beispielsweise unter Verwendung von Fritten. Der Sauerstoff kann jedoch auch unter Verwendung geeigneter Rührer durch kräftiges Rühren in das Reaktionsgemisch eingezogen werden. Die Begasungsintensität, d.h. die pro Zeiteinheit zur Verfügung gestellte Sauerstoffmenge kann ebenfalls stark variieren und richtet sich beispielsweise nach der Wärmeabfuhr und nach der Reaktionsfreudigkeit des eingesetzten o-Kresols. Die maximale Sauerstoffaufnahme und die eventuell davon abweichenden günstigsten Reaktionsbedingungen lassen sich durch einfache Vorversuche ermitteln. Im allgemeinen wird mit Vorteil unterhalb der maximalen Sauerstoffaufnahmefähigkeit des Reaktionsgemisches gearbeitet.

Die Reaktionstemperatur kann in weiten Grenzen schwanken, beispielsweise von 0-200 °C, bevorzugt 20-150 °C.

Zur Aufarbeitung des Reaktionsgemisches nach Beendigung der Sauerstoffaufnahme werden zunächst der Katalysator und gegebenenfalls der Co-Katalysator oder ein Gemisch mehrerer von ihnen abgetrennt, beispielsweise durch Filtrieren, Zentrifugieren oder Abdekantieren. Katalysatoren und Co-Katalysatoren können nach ihrer Abtrennung in einem Folgeansatz wiederverwendet werden. Auch nicht umgesetztes o-Kresol und die Alkohole (VII) und gegebenenfalls die Ether (VIII) werden in einen Folgeansatz zurückgeführt.

Als Kombination für vorteilhalte Reaktionsbedingungen sei genannt: Methanol als Lösungsmittel, Natriumhydroxid, Natriummethanolat, Kaliumhydroxid oder ein Gemisch von diesen als basisch reagierender Stoff, Eisen- und/oder Mangan-tetrakis-(2,4-dimethoxyphenyl)-porphin-chlorid als Katalysator und $Cu(NO_3)_2 \cdot 3H_2O$ als Co-Katalysator.

Der erfindungsgemäß erhaltene o-Hydroxy-benzaldehyd liegt im Reaktionsgemisch als Metallsalz, vielfach als Natrium- oder Kaliumsalz vor. Dieses Salz kann im Lösungsmittel schwer löslich sein, so daß es sich als Niederschlag abscheidet. In diesem Falle erfolgt die Aufarbeitung so, daß dieses Metallsalz des o-Hydroxy-benzaldehydes, gegebenenfalls gemeinsam mit dem Katalysator und dem Co-Katalysator durch Filtration, Zentrifugieren oder Abdekantieren abgetrennt wird. Durch Ansäuern wird sodann der o-Hydroxy-benzaldehyd freigesetzt und kann nach üblichen Methoden isoliert und gegebenenfalls gereinigt werden. Das Filtrat aus der Abtrennung des niedergeschlagenen Salzes wird auf nicht umgesetztes Kresol sowie auf die Alkohole (VII) bzw. Ether (VIII) aufgearbeitet. Deren Wiedereinsatz erfolgt in der beschriebenen Weise.

Eine weitere günstige Aufarbeitungsvariante besteht darin, daß man das Reaktionsgemisch durch Versprühen trocknet, die löslichen Bestandteile des trockenen Rückstandes in heißem Wasser löst und sodann gegebenenfalls die Katalysatoren abfiltriert Für den Fall, daß das Natrium- oder Kaliumsalz des o-Hydroxy-benzaldehydes in kaltem Wasser schlecht löslich ist, kann eine solche wäßrige Lösung auf 50-90 °C erwärmt und bei dieser Temperatur filtriert werden. Aus der wäßrig-alkalischen Lösung wird durch Ansäuern der o-Hydroxy-benzaldehyd freigesetzt und gegebenenfalls vom nicht umgesetzten o-Kresol bzw. von den Alkoholen (VII) und gegebenenfalls den Ethern (VIII) abgetrennt. In bekannter Weise kann die Abtrennung und Reinigung des o-Hydroxy-benzaldehydes nach bekannten Methoden, beispielsweise durch fraktionierte Destillation oder Wasserdampfdestillation oder über die Adduktbildung mit Bisulfit erfolgen.

Für den genannten Fall der Unlöslichkeit des Natrium- oder Kaliumsalzes des o-Hydroxybenzaldehyds in wäßrigem Alkali kann die Isolierung des o-Hydroxybenzaldehyds aus der wäßrig-alkalischen Lösung auch so erfolgen, daß das Natrium- bzw. Kaliumsalz des o-Hydroxybenzaldehyds beispielsweise durch Filtration abgetrennt wird. Aus dem isolierten Natrium- bzw. Kaliumsalz des o-Hydroxybenzaldehyds wird dann durch Ansäuern der o-Hydroxybenzaldehyd freigesetzt. Das Filtrat aus der Abtrennung des niedergeschlagenen Salzes wird wiederum auf nichtumgesetztes Kresol sowie auf die Alkohole (VII) bzw. die Ether (VIII) aufgearbeitet.

In einer noch weiteren Aufarbeitungsvariante wird das Reaktionsgemisch, insbesondere, wenn ein alkoholisches Lösungsmittel als Reaktionsmedium benutzt wurde, mit der 0,5-5-fachen Gewichtsmenge Wasser, bezogen auf das Lösungsmittel, versetzt und das Lösungsmittel mit Hilfe einer Kolonne abdestilliert. Der wäßrig-alkalische Destillationsrückstand wird dann in der beschriebenen Weise weiter aufgearbeitet.

Beispiel 1

In einem 1 l-Reaktor wurde in ein Gemisch aus 54,0 g (0,5 mol) 2-Kresol, 250 g Methanol, 100 g (2,5 mol) Natriumhydroxid, 66 mg (0,07 mmol) Eisen-tetrakis-(2,4-dimethoxyphenyl)-porphin-chlorid und 50 mg (0,2 mmol) $Cu(NO_3)_2.3H_2O$ bei 70 °C unter kräftigem Rühren während 30 h ein Sauerstoffstrom von 1,0 l/h bei Normaldruck eingeleitet.

Anschließend wurde das Methanol abdestilliert, der Rückstand in Wasser aufgenommen und aus der resultierenden wäßrig-alkalischen Lösung die Produktzusammensetzung durch HPLC-Analyse ermittelt.

| Ausbeute (% der theoretischen Ausbeute): | |
|---|---|
| 2-Hydroxybenzaldehyd | 78,3 % |
| 2-Hydroxybenzylmethylether | 6,5% |
| 2-Hydroxybenzylalkohol | nicht gefunden |
| 2-Kresol | nicht gefunden |

Zur Produktisolierung wurde die wäßrig-alkalische Lösung auf pH = 2,5 angesäuert und dreimal mit Ethylacetat extrahiert. Die Ethylacetatphasen wurden vereinigt, über Natriumsulfat getrocknet und das Lösungsmittel am Rotationsverdampfer abgezogen. Als Rückstand blieben 72,8 g einer braungefärbten Flüssigkeit mit einem Gehalt von 65,1 % 2-Hydroxybenzaldehyd und 4,2 % 2-Hydroxybenzylmethylether.

Beispiel 2

In einem 1 l-Reaktor wurde in ein Gemisch aus 54,0 g (0,5 mol) 2-Kresol, 250 g Methanol, 100 g (2,5 mol) Natriumhydroxid und 132 mg (0,14 mmol) Eisen-tetrakis-(2,4-dimethoxyphenyl)-porphin-chlorid bei 70 °C unter kräftigem Rühren wahrend 13h ein Sauerstoffstrom von 2,0 l/h bei Normaldruck eingeleitet.

Die Aufarbeitung erfolgte wie in Beispiel 1.

| Ausbeute (% der theoretischen Ausbeute) in der wäßrig-alkalischen Lösung: | |
|---|---|
| 2-Hydroxybenzaldehyd | 39,6% |
| 2-Hydroxybenzylmethylether | 14,2% |
| 2-Hydroxybenzylalkohol | nicht gefunden |
| 2-Kresol | nicht gefunden |

Beispiel 3

Durchführung wie Beispiel 2 mit dem Unterschied, daß als Katalysator 108 mg Eisen-tetrakis-(2-hydroxyphenyl)-porphin-chlorid eingesetzt wurde.

| Ausbeute (% der theoretischen Ausbeute) in der wäßrig-alkalischen Lösung: | |
|---|---|
| 2-Hydroxybenzaldehyd | 60,3 % |
| 2-Hydroxybenzylmethylether | 13,9 % |
| 2-Hydroxybenzylalkohol | 6.2 % |
| 2-Kresol | nicht gefunden |

Beispiel 4

Durchführung wie Beispiel 2 mit dem Unterschied, daß in Gegenwart von 132 mg (0,14 mmol) Mangan-tetrakis(2,4-dimethoxyphenyl)-porphin-chlorid und 50 mg (0,2 mmol) $Cu(NO_3)_2.3H_2O$ als Katalysatoren gearbeitet wurde.

| Ausbeute (% der theoretischen Ausbeute) in der wäßrig-alkalischen Lösung: | |
|---|---|
| 2-Hydroxybenzaldehyd | 24,9 % |
| 2-Hydroxybenzylmethylether | 22,3 % |
| 2-Hydroxybenzylalkohol | nicht gefunden |
| 2-Kresol | 21,6% |

Beispiele 5 - 10

Die Durchführung erfolgte wie in Beispiel 2 mit dem Unterschied, daß als Katalysatoren jeweils 0,14 mmol Eisen-tetraaryl-porphin-chlorid mit den in der Tabelle 1 spezifizierten Tetrrarylporphinen zusammen mit 50 mg (0,2 mmol) $Cu(NO_3)_2.3H_2O$ eingesetzt wurden. Die Ergebnisse sind in der Tabelle 1 zusammengefaßt.

Tabelle 1 (Beispiele 5-10)

| Nr. | Tetraarylporphin | Ausbeute (% d. th. Ausbeute) | | | |
|---|---|---|---|---|---|
| | | A | B | C | D |
| 5 | Tetrakis-(2-methoxyphenyl)-porphin | 42,2 | 24,9 | 11,6 | 4,2 |
| 6 | Tetrakis-(2,3-dimethoxyphenyl)-porphin | 44,2 | 27,1 | - | - |
| 7 | Tetrakis-(2-ethoxyphenyl)-porphin | 46,1 | 26,6 | 6,9 | 1,6 |
| 8 | Tetrakis-(o-bromphenyl)-porphin | 24,0 | 23,2 | 12,2 | 11,4 |
| 9 | Tetrakis-[3-methyl-thiophenyl(2)]-porphin | 10,1 | 21,3 | 18,5 | 38,4 |
| 10 | Tetrakis-[8-hydroxy-chinolinyl(7)]-porphin | 46,8 | 21,9 | 13,6 | - |

A = 2-Hydroxy-benzaldehyd;

B = 2-Hydroxybenzyl-methylether;

C = 2-Hydroxy-benzylalkohol;

D = 2-Kresol.

Beispiel 11

In einem 1 l-Reaktor wurde in ein Gemisch aus 73,5g (0,5 mol) 4-Chlor-2-methylphenol (97 %), 250 g Methanol, 100 g (2,5 mol) Natriumhydroxid, 132 mg (0,14 mmol) Eisen-tetrakis-(2,4-dimethoxyphenyl)-porphin-chlorid und 50 mg (0,2 mmol) $Cu(NO_3)_2 . 3H_2O$ bei 70 °C unter kräftigem Rühren während 18 h ein Sauerstoffstrom von 2,0 l/h bei Normaldruck eingeleitet.

Anschließend wurde das Methanol unter vermindertem Druck abdestilliert, der Rückstand in Wasser aufgenommen und aus der resultierenden wäßrig-alkalischen Lösung die Produkzusammensetzung durch

HPLC-Analyse ermittelt.

| Ausbeute (% der theoretischen Ausbeute): | |
|---|---|
| 5-Chlor-2-hydroxybenzaldehyd | 44,2% |
| 5-Chlor-2-hydroxybenzylmethylether | 23,7 % |
| 4-Chlor-2-methylphenol | nicht gefunden |

Beispiel 12

Durchführung wie in Beispiel 9 mit dem Unterschied, daß als 2-Kresolderivat 2,3-Dimethylphenol eingesetzt wurde.

| Ausbeute (% der theoretischen Ausbeute): | |
|---|---|
| 6-Methyl-2-hydroxybenzaldehyd | 32,8 % |
| 6-Methyl-2-hydroxybenzylmethylether | 24,1 % |
| 2,3-Dimethylphenol | nicht gefunden |

**Patentansprüche**

**1.** Verfahren zur Herstellung von o-Hydroxy-benzaldehyden der Formel

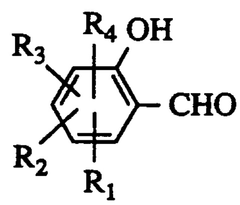

,

in der

$R_1$, $R_2$, $R_3$ und $R_4$ unabhängig voneinander Wasserstoff, geradkettiges oder verzweigtes $C_1$-$C_{10}$-Alkyl, $C_3$-$C_8$-Cycloalkyl, geradkettiges oder verzweigtes $C_1$-$C_{10}$-Alkoxy, Phenyl oder Halogen bedeuten, wobei in p-Stellung zur Hydroxylgruppe Alkyl oder Cycloalkyl nur stehen dürfen, wenn sie, keine α-H-Atome tragen,

durch katalytische Oxidation von o-Kresolen der Formel

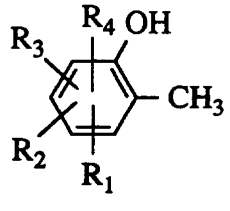

,

in der

$R_1$-$R_4$ die angegebene Bedeutung haben,

mit Sauerstoff in Gegenwart basisch reagierender Stoffe in einem Lösungsmittel, dadurch gekennzeichnet, daß als Katalysatoren Chelatkomplexe des Eisens, Mangans oder eines Gemisches beider mit Polyaza-Makrocyclen als Chelatbildner eingesetzt werden.

2. Verfahren nach Anspruch 1, dadurch gekennzeichnet, daß als Chelatbildner Porphyrine und Porphyrinanaloga eingesetzt werden.

3. Verfahren nach Anspruch 2, dadurch gekennzeichnet, daß als Chelatbildner Phthalocyanine, Porphyrine, Azaporphyrine oder Benzoporphyrine eingesetzt werden.

4. Verfahren nach Anspruch 3, dadurch gekennzeichnet, daß als Chelatbildner meso-Tetraaryl-porphine eingesetzt werden.

5. Verfahren nach Anspruch 4, dadurch gekennzeichnet, daß als Chelatbildner meso-Tetraaryl-porphine eingesetzt werden, deren Arylgruppen mindestens in 2-Stellung substituiert sind.

6. Verfahren nach Anspruch 1, dadurch gekennzeichnet, daß das molare Verhältnis von Eisen- und/oder Mangankomplex zu o-Kresol 0,000001-0,05:1, bevorzugt 0,00001-0,01:1 beträgt.

7. Verfahren nach Anspruch 1, dadurch gekennzeichnet, daß zum Chelatkomplex Kupfer oder eine Kupferverbindung als Co-Katalysator in einem molaren Verhältnis zum o-Kresol von 0,000001-0,1:1, bevorzugt 0,00001-0,05:1 eingesetzt wird.

8. Verfahren nach Anspruch 1, dadurch gekennzeichnet, daß als basisch reagierende Stoffe einer oder mehrere aus der Gruppe der Hydroxide, Alkoholate und Amine von Alkali- und Erdalkalimetallen und Alkoholate und Amide von Aluminium in einer Menge von 1-10 Basenäquivalenten, bevorzugt 1,5-6 Basenäquivalenten pro Mol o-Kresol eingesetzt werden

9. Verfahren nach Anspruch 1, dadurch gekennzeichnet, daß als Lösungsmittel eines oder mehrere aus der Gruppe der Alkohole, Ether, Kohlenwasserstoffe, Halogenkohlenwasserstoffe, Amine, Amide und Sulfoxide, bevorzugt ein oder mehrere Alkohol(e), besonders bevorzugt Methanol eingesetzt wird.

10. Verfahren nach Anspruch 1, dadurch gekennzeichnet, daß als Katalysator ein Metallkomplex der Formel

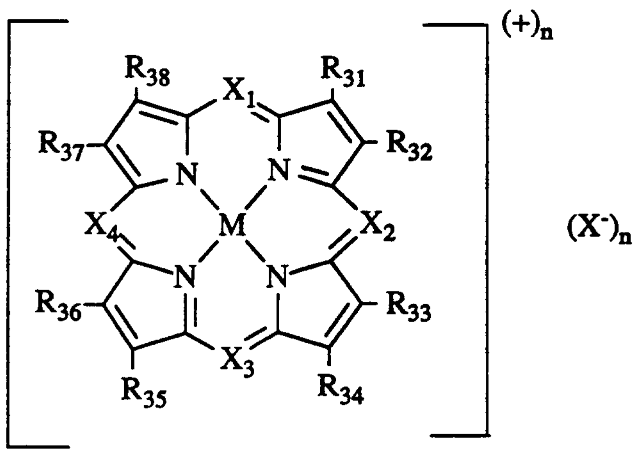

eingesetzt wird, in der

| | |
|---|---|
| M | Eisen oder Mangan bedeutet, |
| $X_1$, $X_2$, $X_3$ und $X_4$ | unabhängig für N oder für das mit dem gleichen Index gekennzeichnete $CY_1$, $CY_2$, $CY_3$ bzw. $CY_4$ stehen, wobei $Y_1$, $Y_2$, $Y_3$ und $Y_4$ unabhängig voneinander Wasserstoff, geradkettiges oder verzweigtes $C_1$-$C_{10}$-Alkyl, $C_3$-$C_8$-Cycloalkyl, $C_7$-$C_{10}$-Aralkyl, $C_6$-$C_{14}$-Aryl, welches teilhydriert sein kann, oder ein aromatisches oder nicht aromatisches 5- oder 6-gliedriges heterocyclisches Ringsystem mit 1 bis 3 Heteroatomen aus der Gruppe N, O und S bedeuten, |

$R_{31}$, $R_{32}$, $R_{33}$, $R_{34}$, $R_{35}$, $R_{36}$, $R_{37}$ und $R_{38}$ unabhängig voneinander Wasserstoff, Halogen, geradkettiges oder verzweigtes $C_1$-$C_{10}$-Alkyl, $C_3$-$C_8$-Cycloalkyl, $C_7$-$C_{10}$-Aralkyl oder Phenyl bedeuten, wobei die beiden Reste eines Pyrrolringes gemeinsam mit den durch sie substituierten C-Atomen $C_6$-$C_{14}$-Aryl, welches teilhydriert sein kann, oder ein aromatisches oder nicht aromatisches 5- oder 6-gliedriges heterocyclisches Ringsystem mit 1 bis 3 Heteroatomen aus der Gruppe N, O und S bilden können,

$X^-$ ein Anionäquivalent einer anorganischen oder organischen Säure oder Hydroxyl oder Alkoxy ist und

n die Zahl Null oder Eins bedeutet.

## Claims

1. Process for the preparation of o-hydroxybenzaldehydes of the formula

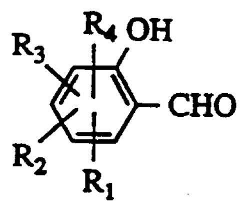

in which

$R_1$, $R_2$, $R_3$ and $R_4$ independently of one another denote hydrogen, straight-chain or branched $C_1$-$C_{10}$-alkyl, $C_3$-$C_8$-cycloalkyl, straight-chain or branched $C_1$-$C_{10}$-alkoxy, phenyl or halogen, where alkyl or cycloalkyl may only be in the p-position to the hydroxyl group if they carry no $\alpha$-H-atoms,

by catalytic oxidation of o-cresols of the formula

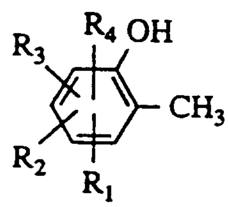

in which

$R_1$-$R_4$ have the meaning indicated,

with oxygen in the presence of substances having a basic reaction and in a solvent has been found, which is characterized in that chelate complexes or iron, manganese or a mixture of both with polyazamacrocycles as chelating agents are employed as catalysts.

2. Process according to Claim 1, characterized in that porphyrins and porphyrin analogues are employed as chelating agents.

3. Process according to Claim 2, characterized in that phthalocyanines, porphyrins, azaporphyrins or benzoporphyrins are employed as chelating agents.

4. Process according to Claim 3, characterized in that meso-tetraaryl-porphines are employed as chelating agents.

**5.** Process according to Claim 4, characterized in that meso-tetraaryl-porphines whose aryl groups are substituted at least in the 2- position are employed as chelating agents.

**6.** Process according to Claim 1, characterized in that the molar ratio of iron and/or manganese complex to o-cresol is 0.000001-0.05:1, preferably 0.00001-0.01:1.

**7.** Process according to Claim 1, characterized in that copper or a copper compound is employed as a co-catalyst to the chelate complex in a molar ratio to the o-cresol of 0.000001-0.1:1, preferably 0.00001-0.05:1.

**8.** Process according to Claim 1, characterized in that as substances having a basic reaction, one or more from the group comprising the hydroxides, alkoxides and amides of alkali metal and alkaline earth metals and alkoxides and amides of aluminium are employed in an amount of 1-10 base equivalents, preferably 1.5-6 base equivalents, per mol of o-cresol.

**9.** Process according to Claim 1, characterized in that, as solvents, one or more is employed from the group comprising the alcohols, ethers, hydrocarbons, halogenohydrocarbons, amines, amides and sulphoxides, preferably one or more alcohol(s), particularly preferably methanol.

**10.** Process according to Claim 1, characterized in that, as a catalyst, a metal complex of the formula

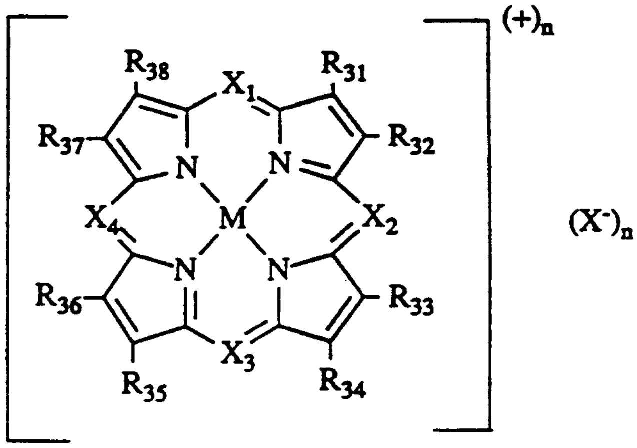

is employed in which

| | |
|---|---|
| M | denotes iron or manganese |
| $X_1$, $X_2$, $X_3$ and $X_4$ | independently represent N or the $CY_1$, $CY_2$, $CY_3$ and $CY_4$ labelled with the same index, where $Y_1$, $Y_2$, $Y_3$ and $Y_4$ independently of one another denote hydrogen, straight-chain or branched $C_1$-$C_{10}$-alkyl, $C_3$-$C_8$-cycloalkyl, $C_7$-$C_{10}$-aralkyl, $C_6$-$C_{14}$-aryl, which can be partially hydrogenated, or an aromatic or non-aromatic 5- or 6-membered heterocyclic ring system having 1 to 3 heteroatoms from the group comprising N, O and S, |
| $R_{31}$, $R_{32}$, $R_{33}$, $R_{34}$, $R_{35}$, $R_{36}$, $R_{37}$ and $R_{38}$ | independently of one another denote hydrogen, halogen, straight-chain or branched $C_1$-$C_{10}$-alkyl, $C_3$-$C_8$-cycloalkyl, $C_7$-$C_{10}$-aralkyl or phenyl, where the two radicals of a pyrrole ring together with the C atoms substituted by them can form $C_6$-$C_{14}$-aryl, which can be partially hydrogenated, or an aromatic or non-aromatic 5- or 6-membered heterocyclic ring system having 1 to 3 heteroatoms from the group comprising N, O and S, |

| | |
|---|---|
| $X^-$ | is an anion equivalent of an inorganic or organic acid, or hydroxyl or alkoxy and |
| n | denotes the number zero or one. |

## Revendications

**1.** Procédé de préparation des o-hydroxybenzaldéhydes de formule

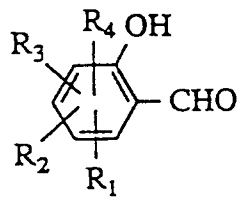

,

dans laquelle

$R_1$, $R_2$, $R_3$ et $R_4$ représentent chacun, indépendamment les uns des autres, l'hydrogène, un groupe alkyle à chaîne droite ou ramifiée en $C_1$-$C_{10}$, un groupe cycloalkyle en $C_3$-$C_8$, un groupe alcoxy à chaîne droite ou ramifiée en $C_1$-$C_{10}$, un groupe phényle ou un halogène, la position para du groupe hydroxy ne pouvant porter que des groupes alkyle ou cycloalkyle qui ne contiennent pas d'atomes d'hydrogène en position alpha,

par oxydation catalytique des o-crésols de formule

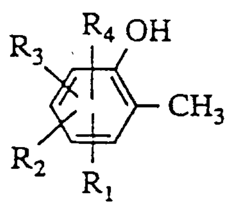

,

dans laquelle $R_1$ à $R_4$ ont les significations indiquées ci-dessus,
par l'oxygène en présence de substances à réaction basique dans un solvant, caractérisé en ce que l'on utilise en tant que catalyseurs des complexes du fer, du manganèse ou d'un mélange de ces deux métaux et de composés polyaza-macrocycliques qui sont les agents complexants.

**2.** Procédé selon la revendication 1, caractérisé en ce que l'on utilise en tant qu'agents complexants des porphyrines et des analogues de porphyrines.

**3.** Procédé selon la revendication 2, caractérisé en ce que l'on utilise en tant qu'agents complexants des phtalocyanines, des porphyrines, des azaporphyrines ou des benzoporphyrines.

**4.** Procédé selon la revendication 3, caractérisé en ce que l'on utilise en tant qu'agents complexants des méso-tétraaryl-porphines.

**5.** Procédé selon la revendication 4, caractérisé en ce que l'on utilise en tant qu'agents complexants des méso-tétraaryl-porphines dont les groupes aryle sont substitués au moins en position 2.

**6.** Procédé selon la revendication 1, caractérisé en ce que le rapport molaire complexe de fer et/ou de manganèse/o-crésol va de 0,000001 à 0,05 : 1, de préférence de 0,00001 à 0,01 : 1.

**7.** Procédé selon la revendication 1, caractérisé en ce que, avec le complexe, on utilise du cuivre ou un dérivé du cuivre en tant que catalyseur auxiliare, à un rapport molaire de 0,000001 à 0,05 : 1, de préférence de 0,00001 à 0,05 : 1 avec l'o-crésol.

**8.** Procédé selon la revendication 1, caractérisé en ce que l'on utilise en tant que substances à réaction basique une ou plusieurs substances prises parmi les hydroxydes, les alcoolates et les amidures de métaux alcalins et alcalino-terreux et les alcoolates et amidures de l'aluminium, en quantités de 1 à 10 équivalents de base, de préférence de 1,5 à 6 équivalents de base par mole d'o-crésol.

**9.** Procédé selon la revendication 1, caractérisé en ce que l'on utilise un ou plusieurs solvants choisis parmi les alcools, les éthers, les hydrocarbures, les hydrocarbures halogénés, les amines, les amides et les sulfoxydes, de préférence un ou plusieurs alcools et tout spécialement le méthanol.

**10.** Procédé selon la revendication 1, caractérisé en ce que l'on utilise en tant que catalyseur un complexe métallique de formule

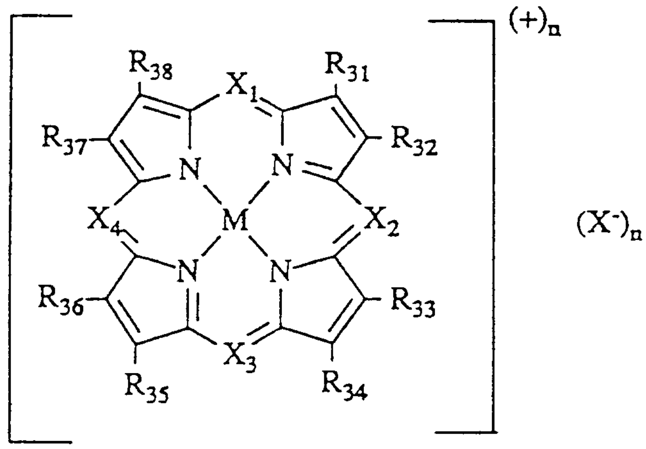

dans laquelle

| | |
|---|---|
| M | représente le fer ou le manganèse, |
| $X_1$, $X_2$, $X_3$ et $X_4$ | représentent chacun, indépendamment les uns des autres, N ou le groupe $CY_1$, $CY_2$, $CY_3$ ou $CY_4$ portant le même indice, $Y_1$, $Y_2$, $Y_3$ et $Y_4$ représentant chacun, indépendamment les uns des autres, l'hydrogène, un groupe alkyle à chaîne droite ou ramifiée en $C_1$-$C_{10}$, un groupe cycloalkyle en $C_3$-$C_8$, un groupe aralkyle en $C_7$-$C_{10}$, un groupe aryle en $C_6$-$C_{14}$ qui peut être hydrogéné en partie, ou un système hétérocyclique aromatique ou non aromatique à 5 ou 6 chaînons contenant 1 à 3 hétéroatomes choisis parmi N, O et S, |
| $R_{31}$, $R_{32}$, $R_{33}$, $R_{34}$, $R_{35}$, $R_{36}$, $R_{37}$ et $R_{38}$ | représentent chacun, indépendamment des uns des autres, l'hydrogène, un halogène, un groupe alkyle à chaîne droite ou ramifiée en $C_1$-$C_{10}$, un groupe cycloalkyle en $C_3$-$C_8$, un groupe aralkyle en $C_7$-$C_{10}$ ou un groupe phényle, les deux substituants d'un cycle pyrrole pouvant former, ensemble et avec les atomes de carbone dont ils sont substituants, un groupe aryle en $C_6$-$C_{14}$ qui peut être partiellement hydrogéné ou un système hétérocyclique aromatique ou non aromatique à 5 ou 6 chaînons contenant 1 à 3 hétéroatomes choisis parmi N, O et S, |
| $X^-$ | représente un équivalent d'un anion d'un acide minéral ou organique, ou un groupe hydroxy ou alcoxy, et |
| n | est égal à 0 ou 1. |